# EUROPEAN PATENT SPECIFICATION

(11) **EP 1 806 152 B1**
(45) Date of publication and mention of the grant of the patent: **01.08.2012**
(21) Application number: 05806193.8
(22) Date of filing: 09.11.2005
(51) Int. Cl.: A61K 47/34, A61K 9/70, A61K 31/192, A61K 31/196, A61K 31/381, A61K 31/405, A61K 31/415, A61K 31/42, A61K 31/5415, A61K 31/63, A61K 45/00, A61K 47/02, A61K 47/14, A61K 47/18, A61K 47/32

(54) **Drug for external use and adhesive patch**
Medikament zur äusseren Anwendung und selbstklebender Verband
Médicament à usage externe et patch adhésif

(30) Priority: 10.11.2004 JP 2004326948
(43) Date of publication of application: 11.07.2007
(73) Proprietor: Hisamitsu Pharmaceutical Co., Inc., Tosu-shi, Saga 841-0017 (JP)
(72) Inventor: SAEKI, Masakazu, Saga 841-0017 (JP); YOSHINAGA, Takaaki, Saga 841-0017 (JP); WAKAMATSU, Masato, Saga 841-0017 (JP)
(74) Representative: Grünecker, Kinkeldey, Stockmair & Schwanhäusser
(86) International application number: PCT/JP2005/020545
(87) International publication number: WO 2006/051818

(56) References cited:
- EP-A1- 1 170 020
- JP-A- 11 349 470
- JP-A- 2001 172 155
- JP-A- 2002 020 274
- JP-A- 2002 193 740
- JP-A- 2004 175 670
- JP-A- 2004 175 670
- JP-A- 2005 320 282

## Description

### Technical Field

The present invention relates to an external preparation and an adhesive patch comprising pharmacologically active components such as anti-inflammatory drugs as active ingredients.

### Background Art

Hitherto known external preparations comprising anti-inflammatory drugs such as non-steroidal anti-inflammatory analgesics as active ingredients include adhesive patches, of the type that contains an anti-inflammatory drug dissolved or dispersed in an aqueous base, in combination with a hydrophilic nonionic surfactant or the like to prevent precipitation of the anti-inflammatory drug (Patent documents 1 and 2).

In the fields of foods and cosmetics, polyglycerin fatty acid esters are used as surfactants with high emulsifying power (Patent documents 3 and 4). Their use has also become known in recent years in the field of external preparations, as solubilizing agents or emulsifying agents for dissolution or dispersion of drugs in aqueous bases, or as penetration accelerators for improved percutaneous absorption of drugs (Patent documents 5 and 6).

For adhesive patches containing indomethacin and L-menthol, a technique is known for including a component such as polyoxyalkylene glycol in order to reduce irritation to skin (Patent document 7).
[Patent document 1] Japanese Unexamined Patent Application Publication No. 2002-20274
[Patent document 2] European Patent Application No. 1170020
[Patent document 3] Japanese Patent No. 3549612
[Patent document 4] Japanese Unexamined Patent Application Publication No. 2004-175670
[Patent document 5] Japanese Unexamined Patent Application Publication No. 2001-131089
[Patent document 6] Japanese Unexamined Patent Application Publication No. 2004-250330
[Patent document 7] Japanese Patent No. 3542814

### Disclosure of the Invention

### Problems to be Solved by the Invention

Conventional external preparations containing pharmacologically active components such as anti-inflammatory drugs, however, have been associated with inconveniences including skin rashes such as redness or edema due to irritation by the pharmacologically active components themselves. Prolonged skin contact by adhesive patches containing high concentrations of active ingredients has been a noted cause of skin rash.

It is therefore an object of the present invention to provide an external preparation comprising a pharmacologically active component such as an anti-inflammatory drug as the active ingredient, whereby skin rash can be adequately prevented.

### Means for Solving the Problems

In order to solve the problem described above, the external preparation of the invention is characterized by containing a pharmacologically active component and a lipophilic polyglycerin fatty acid ester. The pharmacologically active component is preferably an anti-inflammatory drug.

Combination of the lipophilic polyglycerin fatty acid ester with the pharmacologically active component in the external preparation of the invention adequately inhibits skin rash. It has been known in the conventional art that using hydrophilic nonionic surfactants in anti-inflammatory drugs employing aqueous bases inhibits crystallization or precipitation of poorly water-soluble anti-inflammatory drugs. The present inventors have found, however, that using a lipophilic polyglycerin fatty acid ester as a skin irritation-reducing agent can inhibit skin rash caused by irritation by the pharmacologically active components such as anti-inflammatory drugs, and have completed the present invention on the basis of this finding.

The HLB value of the polyglycerin fatty acid ester is preferably no greater than 9. A polyglycerin fatty acid ester having an HLB value within this range will have satisfactory hydrophilicity with the other components in the external preparation, thereby improving the physical properties of the preparation.

In order to more effectively prevent skin rash, the polyglycerin fatty acid ester is preferably an ester of a polyglycerin with an average polymerization degree of 2 to 14 and a C 14-24 fatty acid.

Alternatively, the polyglycerin fatty acid ester is preferably an ester of a polyglycerin and a condensation product of hydroxyl group-containing fatty acids. Such a condensation product is obtained by condensing a plurality of hydroxyl group-containing fatty acids by ester bonding between the hydroxyl and the carboxyl groups, and it has carboxyl groups for ester bonding with the polyglycerin.

In order to more effectively prevent skin rash, the external preparation preferably contains the polyglycerin fatty acid ester at 0.01 to 10 wt% based on the total weight.

The anti-inflammatory drug is preferably at least one selected from the group consisting of non-steroidal anti-inflammatory analgesics, disease modifying antirheumatic drugs and cytokine blockers. The non-steroidal anti-inflammatory analgesic is preferably at least one selected from the group consisting of indomethacin, ketoprofen, diclofenac, flurbiprofen, felbinac, ibuprofen, suprofen, tiaprofen, loxoprofen, celecoxib, rofecoxib, meloxicam and valdecoxib. The external preparation of the invention is particularly useful when using these non-steroidal anti-inflammatory analgesics.

The external preparation of the invention preferably further comprises either or both an edetic acid salt and/or aluminum hydroxide. This will further increase the effect of preventing skin rash by a synergistic effect with the polyglycerin fatty acid ester.

The external preparation of the invention preferably contains substantially no water. This will generally inhibit crystallization or precipitation of poorly water-soluble pharmacologically active components, thereby improving the shelf-life of the external preparation while also allowing a higher concentration of the pharmacologically active component in the external preparation.

The external preparation of the invention preferably further comprises a base for dissolution or dispersion of the pharmacologically active component and polyglycerin fatty acid ester. The base preferably contains one or more substances selected from the group consisting of natural rubber, synthetic isoprene rubber, polyisobutylene, polyvinyl ether, polyurethane, polyisoprene, polybutadiene, styrene-butadiene copolymer, styrene-isoprene copolymer, styrene-isoprene-styrene block copolymer, acrylic pressure-sensitive adhesives and silicone-based pressure-sensitive adhesives. This can impart sufficient adhesion to the external preparation for prolonged attachment.

The invention further provides an adhesive patch comprising: a pressure-sensitive adhesive layer including the external preparation; and a backing bearing the pressure-sensitive adhesive layer. The external preparation of the invention is particularly useful for an adhesive patch because an adhesive patch is usually in contact with affected areas for prolonged periods and tends to cause skin rash.

### Effect of the Invention

According to the present invention there is provided an external preparation comprising a pharmacologically active component such as an anti-inflammatory drug as the active ingredient, whereby skin rash can be adequately prevented.

### Brief Description of the Drawings

Fig. 1 is a perspective view of an embodiment of the adhesive patch of the invention.
Fig. 2 is a graph showing the results of a carrageenan foot edema test carried out for the examples.
Fig. 3 is a graph showing the results of a yeast inflammation foot pain test carried out for the examples.

### Explanation of Symbols

1: Adhesive patch, 2: backing, 3: pressure-sensitive adhesive layer, 4: release liner.

### Best Mode for Carrying Out the Invention

Preferred embodiments of the invention will now be described in detail.

The external preparation of the invention contains at least a pharmacologically active component and a lipophilic polyglycerin fatty acid ester. The pharmacologically active component is preferably an anti-inflammatory drug, and as anti-inflammatory drugs there may be mentioned non-steroidal anti-inflammatory analgesics, disease modifying antirheumatic drugs (DMARD), cytokine blockers and the like. As suitable pharmacologically active components other than anti-inflammatory drugs there may be mentioned vasodilators, anti-arrhythmia drugs, antihypertensive agents, antitussive expectorants, antidementia drugs, antianxiety drugs, dysuria treatment agents, antidepressants, neurosis drugs, antiplatelet drugs, antiviral agents, antitumor agents, local anesthetics, hormone agents, antihistamines, anti-coagulants, antispastic drugs, general anesthetics, hypnotic/analgesics, anti-epileptic agents, stimulant/analeptics, antimotion sickness agents, psychoneurotic drugs, skeletal muscle relaxants, autonomic nerve agents, anti-Parkinson drugs, diuretics, vasoconstrictors, respiratory stimulants, peptic ulcer agents, cholagogues, urogenital/anal agents, agents for parasitic skin diseases, emollients, vitamins, antifungal agents, inorganic formulations, hemostatic drugs, hepatic disease drugs, habitual addiction drugs, gout treatment agents, diabetes treatment agents, antibiotics, chemotherapeutic agents, narcotics, smoking cessation aids and angina drugs.

As non-steroidal anti-inflammatory analgesics there may be mentioned ketoprofen, loxoprofen, ibuprofen, flurbiprofen, tiaprofen, indomethacin, acemetacin, diclofenac, felbinac, sulindac, etodolac, tolmetin, piroxicam, meloxicam, ampiroxicam, naproxen, azapropazone, methyl salicylate, glycol salicylate, valdecoxib, celecoxib, rofecoxib and the like, any of which may be used alone or in combinations of different types. Especially preferred among these from the viewpoint of superior pharmacological effects are indomethacin, ketoprofen, diclofenac, flurbiprofen, felbinac, ibuprofen, suprofen, tiaprofen and loxoprofen. As disease modifying antirheumatic drugs there may be mentioned leflunomide and auranofin, and as cytokine blockers there may be mentioned infliximab, etanercept, adalimubab and anakinra.

As vasodilators there may be mentioned diltiazem hydrochloride, pentaerythritol tetranitrate, isosorbide nitrate, trapidil, nicorandil, prenylamine lactate, molsidomine, aluminum nitrite, tolazoline hydrochloride and nifedipine. As anti-arrhythmia drugs there may be mentioned procainamide hydrochloride, lidocaine hydrochloride, propranolol hydrochloride, alprenolol hydrochloride, atenolol, nadolol, metoprolol tartrate, ajmaline, disopyramide and mexiletine hydrochloride. As antihypertensive agents there may be mentioned ecarazine hydrochloride, indapamide, clonidine hydrochloride, bisoprolol fumarate, bunitrolol hydrochloride, labetalol hydrochloride, capropril, guanabenz acetate, mebutamate and betanidine sulfate. As antitussive expectorants there may be mentioned carbetapentane citrate, chloperastine, oxeladin tannate, clobutinol hydrochloride, clofedanol hydrochloride, noscapine hydrochloride, ephedrine hydrochloride, isoproterenol hydrochloride, clorprenaline hydrochloride, methoxyphenamine hydrochloride, procaterol hydrochloride, tulobuterol hydrochloride, clenbuterol hydrochloride and ketotifen fumarate. As antidementia drugs there may be mentioned donepezil hydrochloride and the like. As antianxiety drugs there may be mentioned tandospirone citrate and the like. As dysuria treatment agents there may be mentioned oxybutynin hydrochloride and tamsulosin hydrochloride. As antidepressants there may be mentioned fluvoxamine maleate, citalopram, fluoxetine, sertraline hydrochloride and paroxetine hydrochloride. As neurosis drugs there may be mentioned pregabalin and the like. As antiplatelet drugs there may be mentioned sarpogrelate hydrochloride and the like. As antiviral agents there may be mentioned aciclovir and ganciclovir. As antitumor agents there may be mentioned cyclophosphamide, fluorouracil, methotrexate, tegafur, mitomycin C, procarbazine hydrochloride, doxifluridine and ranimustine. As local anesthetics there may be mentioned ethyl aminobenzoate, tetracaine hydrochloride, procaine hydrochloride, dibucaine hydrochloride, oxybuprocaine hydrochloride and protocaine hydrochloride. As hormone agents there may be mentioned propylthiouracil, thiamazole, metolonone acetate, estradiol, estriol and progesterone. As antihistamines there may be mentioned diphenhydramine hydrochloride, chlorpheniramine maleate, promethazine, cyproheptadine hydrochloride and diphenylpyraline hydrochloride. As anti-coagulants there may be mentioned warfarin potassium and ticlopidine hydrochloride. As antispastic drugs there may be mentioned atropine methylbromide and scopolamine. As general anesthetics there may be mentioned thiopental sodium and pentobarbital sodium. As hypnotic/analgesics there may be mentioned bromvalerylurea, amobarbital and phenobarbital. As anti-epileptic agents there may be mentioned phenytoin sodium and the like. As stimulant/analeptics there may be mentioned methamphetamine hydrochloride and the like. As antimotion sickness agents there may be mentioned diphenidol hydrochloride and betahistine mesylate. As psychoneurotic drugs there may be mentioned chlorpromazine hydrochloride, thioridazine, meprobamate, imipramine hydrochloride, chlordiazepoxide and diazepam. As skeletal muscle relaxants there may be mentioned suxamethonium hydrochloride and eperisone hydrochloride. As autonomic nerve agents there may be mentioned neostigmine bromide and bethanechol hydrochloride. As anti-Parkinson drugs there may be mentioned amantadine hydrochloride, pergolide mesylate, bromocriptine mesylate, ropinirole and selegiline. As diuretics there may be mentioned hydroflumethiazide, isosorbide and furosemide. As vasoconstrictors there may be mentioned epinephrine and phenylephrine hydrochloride. As respiratory stimulants there may be mentioned lobeline bromide, dimorpholamine and naloxone hydrochloride. As peptic ulcer agents there may be mentioned glycopyrronium bromide, proglumide, cetraxate hydrochloride, cimetidine and spizofurone. As cholagogues there may be mentioned ursodesoxycholic acid and osalmid. As urogenital/anal agents there may be mentioned hexamine, sparteine, dinoprost and ritodrine hydrochloride. As agents for parasitic skin diseases there may be mentioned salicylic acid, ciclopiroxolamine and croconazole hydrochloride. As emollients there may be mentioned urea and the like. As vitamins there may be mentioned calcitriol, thiamine hydrochloride, riboflavin sodium phosphate, pyridoxine hydrochloride, nicotinic acid amide, panthenol and ascorbic acid. As antifungal agents there may be mentioned bifonazole, clotrimazole, tioconazole, miconazole, econazole, isoconazole, itraconazole, sulconazole, oxiconazole, omoconazole, croconazole, ketoconazole, neticonazole, lanoconazole, fluconazole, terbinafine, naftifine, butenafine and amorolfine. As inorganic formulations there may be mentioned calcium hydrochloride, potassium iodide and sodium iodide. As hemostatic drugs there may be mentioned etamsylate and the like. As hepatic disease drugs there may be mentioned tiopronin and the like. As habitual addiction drugs there may be mentioned cyanamide and the like. As gout treatment agents there may be mentioned colchicine, probenecid and sulfinpyrazone. As diabetes treatment agents there may be mentioned tolbutamide, chlorpropamide, glymidine sodium, glipizole, buformin hydrochloride and insulin. As antibiotics there may be mentioned benzylpenicillin potassium, propicillin potassium, cloxacillin sodium, ampicillin sodium, bacampicillin hydrochloride, carbenicillin sodium, cephaloridine, cephoxitin sodium, erythromycin, chloramphenicol, tetracycline, kanamycin sulfate and cycloserine. As chemotherapeutic agents there may be mentioned isocyanide, pyrazinamide and ethionamide. As narcotics there may be mentioned morphine hydrochloride, codeine phosphate, cocaine hydrochloride, pethidine hydrochloride and fentanyl citrate. As smoking cessation aids there may be mentioned nicotine and the like. As angina drugs there may be mentioned nitroglycerin and the like.

The pharmacologically active components listed above as examples that are free forms of compounds may also be used as pharmaceutically acceptable salts. Also, those listed as salts may be used in their corresponding pharmaceutically acceptable free forms or as other pharmaceutically acceptable salts.

The polyglycerin fatty acid ester has an HLB value of preferably no greater than 9 and more preferably no greater than 7. The HLB value is also preferably 2 or greater.

The polyglycerin fatty acid ester is also preferably an ester of a polyglycerin with an average polymerization degree of 2 to 14 and a C14-24 saturated or unsaturated fatty acid. For further reduced skin irritation, the average polymerization degree of the polyglycerin is more preferably 8 to 10 and the number of carbon atoms of the saturated or unsaturated fatty acid is more preferably 18 to 22.

The polyglycerin fatty acid ester is even more preferably formed by ester bonding between a polyglycerin and the condensation product of two hydroxyl group-containing fatty acids. As hydroxyl group-containing fatty acids there may be mentioned ricinoleic acid and the like, and the product of ester bonding between a polyglycerin and the condensation product obtained by condensation of two ricinoleic acid molecules by ester bonding may be suitably used as the polyglycerin fatty acid ester.

Such a polyglycerin fatty acid ester can be obtained by a process known in the conventional art such as polyglycerin and fatty acid esterification, and commercial products are also available.

The content of the polyglycerin fatty acid ester in the external preparation is preferably 0.01 to 10 wt% and more preferably 1 to 3 wt% based on the total weight. If the polyglycerin fatty acid ester content is less than 0.01 wt% or greater than 10 wt%, the effect of preventing skin rash will tend to be reduced.

The external preparation of the invention preferably further comprises an edetic acid salt, aluminum hydroxide or the like. The synergistic effect of these with the polyglycerin fatty acid ester can further increase the effect of preventing skin rash.

As edetic acid salts there are preferred one or more selected from the group consisting of disodium edetate, trisodium edetate and tetrasodium edetate. From the viewpoint of the effect of reducing skin irritation, the content of edetic acid salts in the external preparation is preferably 0.01 to 5 wt% and more preferably 1 to 3 wt% based on the total weight.

When aluminum hydroxide is used, on the other hand, the content is preferably 0.01 to 10 wt% based on the total weight, from the viewpoint of the effect of reducing skin irritation.

The external preparation of the invention is preferably an oilbased external preparation containing substantially no water. If the external preparation contains substantially no water, precipitation of pharmacologically active components such as poorly water-soluble non-steroidal anti-inflammatory analgesics will be inhibited, thereby stabilizing the properties of the external preparation. The phrase "contains substantially no water" as used herein means that the external preparation is composed essentially of non-aqueous components. However, the external preparation may contain the trace amount of water deriving from the starting materials or the production environment, so long as this produces no inhibition on the effect of the invention of preventing skin rash. More specifically, the water content of the external preparation is preferably no greater than 1.0 wt% based on the total weight of the external preparation.

The external preparation of the invention preferably further contains a base for dissolution or dispersion of the pharmacologically active component (preferably anti-inflammatory drug) and the polyglycerin fatty acid ester.

The base is not particularly restricted so long as it can uniformly disperse or dissolve the components including the pharmacologically active component and polyglycerin fatty acid ester, and for example, rubber bases, acrylic pressure-sensitive adhesives and silicone-based pressure-sensitive adhesives may be used.

As rubber-based pressure-sensitive adhesives there are preferred natural rubber, synthetic isoprene rubber, polyisobutylene, polyvinyl ether, polyurethane, polyisoprene, polybutadiene, styrene-butadiene copolymer, styrene-isoprene copolymer and styrene-isoprene-styrene block copolymer.

As acrylic pressure-sensitive adhesives there are preferred acrylic polymers that are homopolymers or copolymers of (meth)acrylic alkyl esters. The alkyl group of the (meth)acrylic acid ester is preferably C4-18. The term "(meth)acrylic" means "methacrylic or acrylic" (same hereunder).

As specific (meth)acrylic alkyl esters there may be mentioned butyl acrylate, isobutyl acrylate, hexyl acrylate, octyl acrylate, 2-ethylhexyl acrylate, isooctyl acrylate, decyl acrylate, isodecyl acrylate, lauryl acrylate, stearyl acrylate, methyl methacrylate, ethyl methacrylate, butyl methacrylate, isobutyl methacrylate, 2-ethylhexyl methacrylate, isooctyl methacrylate, decyl methacrylate, isodecyl methacrylate, lauryl methacrylate and lauryl methacrylate.

The acrylic polymer may be a copolymer of the aforementioned (meth)acrylic alkyl ester with another monomer. Other suitable monomers include those with hydroxyl groups, carboxyl groups, amide groups, amino groups and pyrrolidone rings. As examples of monomers with hydroxyl groups there may be mentioned hydroxyalkyl (meth)acrylates such as 2-hydroxyethyl (meth)acrylate and hydroxypropyl (meth)acrylate. As examples of monomers with carboxyl groups there may be mentioned α, β-unsaturated carboxylic acids such as acrylic acid and methacrylic acid, monoalkyl maleic acid esters such as butyl maleate, and maleic acid, fumaric acid, crotonic acid and the like. Since maleic anhydride contributes the same copolymerizing component as maleic acid, it may also be used as a monomer. As examples of monomers with amide groups there may be mentioned alkyl(meth)acrylamides such as acrylamide, dimethylacrylamide and diethylacrylamide, N-alkoxymethyl(meth)acrylamides such as N-butoxymethylacrylamide and N-ethoxyethylacrylamide, and diacetoneacrylamide. As monomers with amino groups there may be mentioned dimethylaminoethyl acrylate and the like. As monomers with pyrrolidone rings there may be mentioned N-vinyl-2-pyrrolidone and the like.

As silicone-based pressure-sensitive adhesives there may be mentioned those composed mainly of polyorganosiloxanes such as polydimethylsiloxane.

The external preparation of the invention may also contain, if necessary, pharmaceutically acceptable components other than those mentioned above, such as skin rash inhibitors, saccharides, metal oxides, hydrophilic polymers, humectants, refreshing agents, antioxidants, antiseptic agents, preservatives, aromatics and the like.

The dosage form of the external preparation of the invention is not particularly restricted and may be an adhesive patch, ointment, gel, cream, poultice, suppository, liniment, eye drop, aerosol or the like, but it is preferably an adhesive patch comprising: a pressure sensitive adhesive layer including the aforementioned base-containing external preparation; and a backing bearing the pressure-sensitive adhesive layer.

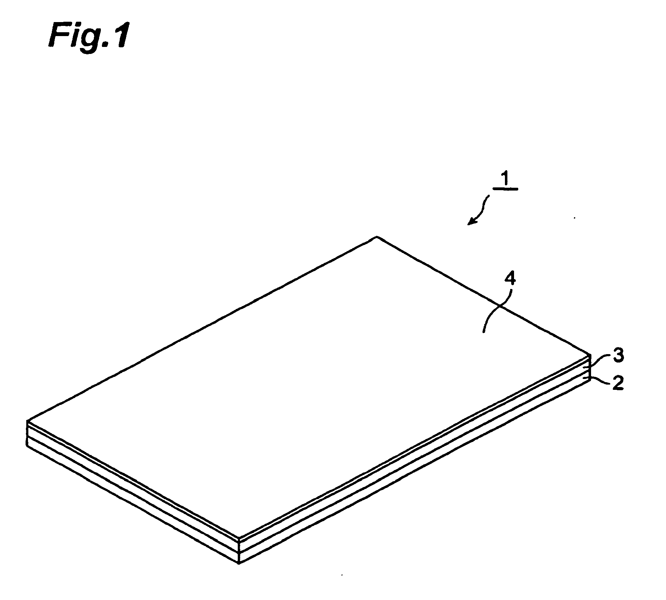

Fig. 1 is a perspective view of an embodiment of an adhesive patch according to the invention. The adhesive patch 1 shown in Fig. 1 has a construction with a pressure-sensitive adhesive layer 3 composed of the external preparation and a release liner 4 laminated in that order on one side of a backing 2. The adhesive patch 1 is used by peeling off the release liner 4 and attaching it to skin with the pressure-sensitive adhesive layer 3 in contact with the skin.

A tackifier, softening agent or the like may also be added to the pressure-sensitive adhesive layer 3. As examples of tackifiers there may be mentioned rosins and rosin derivatives obtained by hydrogenation, disproportionation, polymerization or esterification of rosins, terpene resins such as α-pinene and β-pinene, terpene-phenol resins, aliphatic, aromatic, alicyclic or copolymer petroleum resins, alkylphenol resins, and xylene resins. A softening agent is a component which plasticizes or softens the base polymer to maintain a suitable degree of adhesion onto skin, and as examples there may be mentioned polybutene, liquid paraffins, higher fatty acid esters such as isopropyl myristate, silicone oils, and vegetable oils such as almond oil, camellia oil, persic oil and peanut oil.

The backing 2 used in the adhesive patch may be either elastic or non-elastic, and it is preferably one that allows efficient release of the pharmacologically active component. Specifically, as the backing 2 there may be suitably used a film or sheet formed of a synthetic resin such as polyethylene, polypropylene, polybutadiene, ethylene-vinyl acetate copolymer, polyvinyl chloride, polyester, nylon or polyurethane, or a laminate, porous film, foam, woven fabric or nonwoven fabric composed thereof, or a paper material.

An adhesive patch of this type can be fabricated by a fabrication method known in the conventional art. For example, in the case of a tape preparation employing a rubber-based pressure-sensitive adhesive as the base, first a kneader, mixer or the like is used to combine the base with the other components, such as a softening agent and tackifier, preferably while heating to 120 to 160°C, and then the pharmacologically active component such as an anti-inflammatory drug is added and mixed therewith while heating to a degree that does not cause its thermal decomposition, to prepare a mixture for formation of a pressure-sensitive adhesive layer. The mixture is either directly spread onto a film support to form a pressure-sensitive adhesive layer, or it is spread onto a released-treated paper sheet or film to form a pressure-sensitive adhesive layer, and the backing placed thereover for contact transfer of the pressure-sensitive adhesive layer onto the backing. For an acrylic tape preparation employing an acrylic pressure-sensitive adhesive as the base, a coating solution comprising the base, pharmacologically active component, absorption accelerator, etc. dissolved or dispersed in a solvent may be directly coated onto the surface of the backing and then dried to form a pressure-sensitive adhesive layer. The solvent used in this case is preferably one that dissolves all of the constituent components including the base and pharmacologically active component, and for example, there may be mentioned aromatic hydrocarbons such as toluene, benzene and xylene, esters such as ethyl acetate, and halogenated hydrocarbons such as carbon tetrachloride, chloroform and methylene chloride.

### Examples

The invention will now be explained in greater detail by working examples.

### (Fabrication of adhesive patch)

As examples and comparative examples, mixtures uniformly blended with the formulations (wt%) shown in Tables 1 and 2 were each spread onto a polyester film to a thickness of 100 µm using a spreader to form a pressure-sensitive adhesive layer, a backing was laminated therewith in a manner covering the pressure-sensitive adhesive layer, and the laminate was cut to a prescribed shape to fabricate an adhesive patch. As polyglycerin fatty acid esters there were used: a polyglycerin fatty acid ester A with an HLB value of 3 which was an ester of a polyglycerin with an average polymerization degree of 10 and a C18 fatty acid for Examples 1 to 4, a polyglycerin fatty acid ester B with an HLB of 5 which was an ester of a polyglycerin with an average polymerization degree of 10 and a C22 fatty acid for Examples 5 to 12, and a hydrophilic polyglycerin fatty acid ester C with an HLB value of 17 which was an ester of a polyglycerin with an average polymerization degree of 10 and a C12 fatty acid for Comparative Examples 2 and 3.
For Comparative Example 4 there was prepared a conventional indomethacin-containing adhesive patch (indomethacin content: 3.75 wt%), as a reference example there was prepared a Japanese Pharmacopeia bandage, and as Comparative Example 5 there was prepared a conventional indomethacin-containing cream preparation (indomethacin content: 1.0 wt%, no polyglycerin fatty acid ester).

**[Table 1]**

| | Example | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| Indomethacin | 5.00 | 5 00 | 3.50 I | 3.50 I | 5.00 | 5.00 | 3.50 | 3.50 | 3.50 | 3 50 | 3.50 | 3.50 |
| L-menthol | 5.00 00 | 5.00 | 3.50 | 3.50 | 5.00 | 5.00 | 3.50 | 3.50 | 3.50 | 3.50 | 3.50 | 3.50 |
| Polyglycerin fatty acid ester A | 1.00 | 3.00 | 1.00 | 3.00 | 3.00 | - | - | - | - | - | - | - |
| Polyglycerin fatty acid ester B | - | - | - | - | 1.00 | 3.00 | 1.00 | 3.00 | 1.00 | 3.00 | 1.00 | 1.00 |
| Aluminum hydroxide | - | - | - | - | 1.50 | 1.50 | 1.50 | 1.50 | 1.50 | 1.50 | 1.50 | 1.50 |
| Disodium ederate | - | - | - | - | - | - | - | - | 0.80 | 0.80 | - | 0.80 |
| Dibutylhydroxy Toluene | 1.5 | 1.5 | 1.5 | 1.5 | 1.5 | 1.5 | 1.5 | 1.5 | 1.5 | 1.5 | 1.5 | 1.5 |
| Styrene-isoptene -styrene block copolymer | 19.0 | 19.0 | 19.0 | 19.0 | 19.0 | 19.0 | 19.0 | 19.0 | 19.0 | 19.0 | 19.0 | 19.0 |
| Polyisobutylene | 12.5 | 12.5 | 12.5 | 12.5 | 12.5 | 12.5 | 12.5 | 12.5 | 12.5 | 12.5 | 12.5 | 12.5 |
| Alicyclic saturated h drocarbon resin | 12.5 | 12.5 | 12.5 | 12.5 | 12.5 | 12.5 | 12.5 | 12.5 | 12.5 | 12.5 | 12.5 | 12.5 |
| Titanium oxide | 3.0 | 3.0 | 3.0 | 3.0 | 3.0 | 3.0 | 3.0 | 3.0 | 3.0 | 3.0 | - | - |
| Liquid paraffin | 40.5 | 38.5 | 43.5 | 41.5 | 39.0 | 37.0 | 42.0 | 40.0 | 41.2 | 39.2 | 45.0 | 44.2 |
| Skin irritation index (SI value) | 13.3 | 16.7 | 13.3 | 16.7 | 6.7 | 13.3 | 16.7 | 20.0 | 0 | 10.0 | 12.1 | 5.0 |

**[Table 2]**

| | Comp. Ex. | | | | | Reference Example |
|---|---|---|---|---|---|---|
| | 1 | 2 | 3 | 4 | 5 | |
| Indomethacin | 5.00 | 3.50 | 3.50 | Indomethacin-containing adhesive patch | Indomethacin-containing cream preparation | Japanese Pharmacopeia bandage |
| L-menthol | 5.00 | 3.50 | 3.50 | | | |
| Polyglycerin fatty acid ester C | - | 1.00 | 3.00 | | | |
| Aluminum hydroxide | - | 1.50 | 1.30 | | | |
| Disodium edetate | - | - | - | | | |
| Dibutylhydroxy Toluene | 1.5 | 1.5 | 1.5 | | | |
| Styrene-isoprene -styrene block copolymer | 19.0 | 19.0 | 19.0 | | | |
| Polyisobutylene | 12.5 | 12.5 | 12.5 | | | |
| Alicyclic saturated hydrocarbon resin | 12.5 | 12.5 | 12.5 | | | |
| Titanium oxide | 3.0 | 3.0 | 3.0 | | | |
| Liquid paratifin | 41.5 | 42.0 | 40.0 | | | |
| Skin irritation index (S1 value) | 33.3 | 26.6 | 30.0 | 40.0 | - | 6.7 |

### (48 Hour closed patch test)

Each adhesive patch was used for a 48 hour patch test with 15 healthy adult males, and the skin irritation was evaluated. The test was conducted by a method in which the adhesive patch (1.5 cm-diameter circle) was attached to the back of each person and the skin irritation index (SI value) after 48 hours was determined. The skin irritation index was determined by the Sugai system (see "Skin", Vol. 27, No.4, August 1985). The results are shown in Tables 1 and 2.

### (Carrageenan foot edema test)

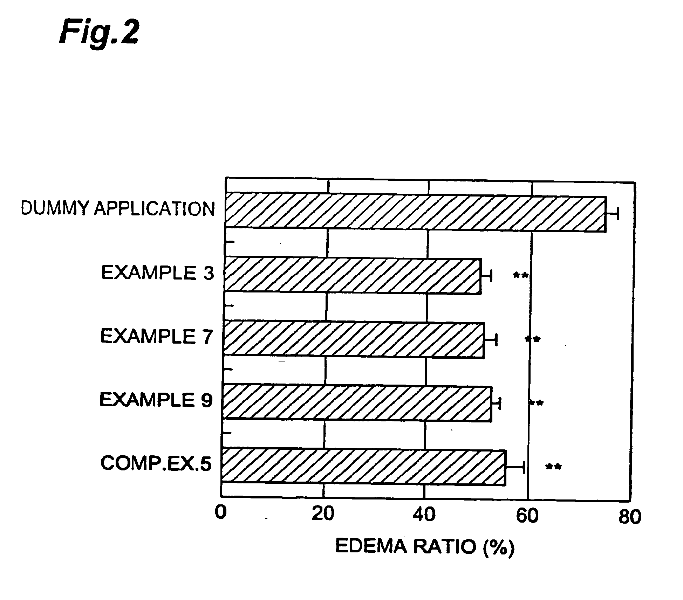

The adhesive patches of Examples 3, 7 and 9 and the cream preparation of Comparative Example 5 were subjected to the carrageenan foot edema test described below.
First, they were applied onto the right hind legs of SD male rats (10 per group) each having a body weight of about 140 g, and the drug was removed 4 hours after application. The adhesive patches of Examples 3, 7 and 9 were 8 cm² in size, and the cream preparation of Comparative Example 5 was applied in an amount of 200 mg. Immediately after removal of the drug, 0.1 mL of a 1.5% carrageenan solution was subcutaneously injected into the footpad to provoke an inflammatory response. The foot volume was measured 3 hours after provoking inflammation, and the ratio with respect to the foot volume prior to injection was calculated as the edema ratio. A dummy application test was also carried out. The results are shown in Fig. 2.

### (Yeast inflammation foot pain test)

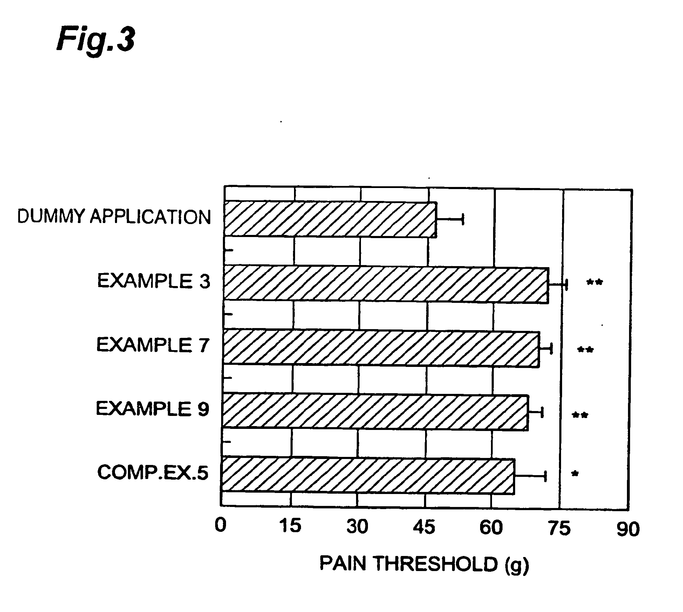

The adhesive patches of Examples 3, 7 and 9 and the cream preparation of Comparative Example 5 were subjected to the yeast inflammation foot pain test described below.
First, they were applied onto the right hind legs of SD male rats (12 per group) each having a body weight of about 130 g, and the drug was removed 4 hours after application. The adhesive patches of Examples 3, 7 and 9 were 8 cm² in size, and the cream preparation of Comparative Example 5 was applied in an amount of 200 mg. Immediately after removal of the drug, 0.2 mL of a 25% yeast solution was subcutaneously injected into the footpad to provoke an inflammatory response. The pain threshold was measured 3 hours after provoking inflammation. A dummy application test was also carried out. The results are shown in Fig. 3.

As shown in Table 1, Fig. 2 and Fig. 3, the adhesive patches of the examples produced lower skin irritation and prevented skin rash, compared to the comparative examples that contained no lipophilic polyglycerin fatty acid ester. It was therefore confirmed that the invention can provide an external preparation that adequately inhibits skin rash.

## Claims

1. An external preparation containing:
an anti-inflammatory drug; and
a lipophilic polyglycerin fatty acid ester,
wherein the polyglycerin fatty acid ester is an ester of a polyglycerin with an average polymerization degree of 2 to 14 and a C14-24 fatty acid; and
wherein the water content of the external preparation is no greater than 1.0 wt% based on the total weight of the external preparation.

2. An external preparation according to claim 1,
wherein the HLB value of the polyglycerin fatty acid ester is no greater than 9.

3. An external preparation according to claim 1 or 2,
wherein the polyglycerin fatty acid ester is an ester of a polyglycerin and a condensation product of hydroxyl group-containing fatty acids.

4. An external preparation according to any one of claims 1 to 3,
which contains the polyglycerin fatty acid ester at 0.01 to 10 wt% based on the total weight.

5. An external preparation according to claim 1,
wherein the anti-inflammatory drug is at least one selected from the group consisting of non-steroidal anti-inflammatory analgesics, disease modifying antirheumatic drugs and cytokine blockers.

6. An external preparation according to claim 5,
wherein the non-steroidal anti-inflammatory analgesic is at least one selected from the group consisting of indomethacin, ketoprofen, diclofenac, flurbiprofen, felbinac, ibuprofen, suprofen, tiaprofen, loxoprofen, celecoxib, rofecoxib, meloxicam and valdecoxib.

7. An external preparation according to any one of claims 1 to 6.
which further contains either or both an edetic acid salt and/or aluminum hydroxide.

8. An external preparation according to any one of claims 1 to 7.
which further contains a base that dissolves or disperses the pharmacologically active component and polyglycerin fatty acid ester.

9. An external preparation according to claim 8,
wherein the base contains at least one substance selected from the group consisting of natural rubber, synthetic isoprene rubber, polyisobutylene, polyvinyl ether, polyurethane, polyisoprene, polybutadiene, styrene-butadiene copolymer, styrene-isoprene copolymer,
styrene-isoprene-styrene block copolymer, acrylic pressure-sensitive adhesives and silicone-based pressure-sensitive adhesives.

10. An adhesive patch comprising:
a pressure-sensitive adhesive layer including an external preparation according to claim 8 or 9; and
a backing bearing the pressure-sensitive adhesive layer.

## Patentansprüche

1. Äußerliche Zubereitung, enthaltend:
ein entzündungshemmendes Arzneimittel; und
einen lipophilen Polyglycerinfettsäureester,
wobei der Polyglycerinfettsäureester ein Ester von einem Polyglycerin mit einem mittleren Polymerisationsgrad von 2 bis 14 und einer C14-24-Fettsäure ist; und
wobei der Wassergehalt der äußerlichen Zubereitung nicht mehr als 1,0 Gew.-%, bezogen auf das Gesamtgewicht der äußerlichen Zubereitung, beträgt.

2. Äußerliche Zubereitung nach Anspruch 1, wobei der HLB-Wert des Polyglycerinfettsäureesters nicht mehr als 9 beträgt.

3. Äußerliche Zubereitung nach Anspruch 1 oder 2,
wobei der Polyglycerinfettsäureester ein Ester von einem Polyglycerin und einem Kondensationsprodukt von eine Hydroxylgruppe enthaltenden Fettsäuren ist.

4. Äußerliche Zubereitung nach einem der Ansprüche 1 bis 3,
welche den Polyglycerinfettsäureester in einer Menge von 0,01 bis 10 Gew.-%, bezogen auf das Gesamtgewicht, enthält.

5. Äußerliche Zubereitung nach Anspruch 1,
wobei das entzündungshemmende Arzneimittel wenigstens eines, ausgewählt aus der Gruppe bestehend aus nicht-steroidalen entzündungshemmenden Analgetika, krankheitsmodifizierenden anti-rheumatischen Arzneimitteln und Cytokinblockern, ist.

6. Äußerliche Zubereitung nach Anspruch 5,
wobei das nicht-steroidale entzündungshemmende Analgetikum wenigstens eines, ausgewählt aus der Gruppe bestehend aus Indomethacin, Ketoprofen, Diclofenac, Flurbiprofen, Felbinac, Ibuprofen, Suprofen, Tiaprofen, Loxoprofen, Celecoxib, Rofecoxib, Meloxicam und Valdecoxib, ist.

7. Äußerliche Zubereitung nach einem der Ansprüche 1 bis 6, welche außerdem eines oder beide von einem Ethylendiamintetraessigsäuresalz und/oder Aluminiumhydroxid enthält.

8. Äußerliche Zubereitung nach einem der Ansprüche 1 bis 7,
welche außerdem eine Grundlage enthält, welche die pharmakologisch aktive Komponente und Polyglycerinfettsäureester löst oder dispergiert.

9. Äußerliche Zubereitung nach Anspruch 8,
worin die Grundlage wenigstens eine Substanz, ausgewählt aus der Gruppe bestehend aus Naturkautschuk, synthetischem Isoprenkautschuk, Polyisobutylen, Polyvinylether, Polyurethan, Polyisopren, Polybutadien, Styrol-Butadien-Copolymer, Styrol-Isopren-Copolymer, Styrol-Isopren-Styrol-Blockcopolymer, Acryl-Haftklebern und Haftklebern auf Siliconbasis, enthält.

10. Heftpflaster umfassend:
eine Haftkleberschicht, die eine äußerliche Zubereitung nach Anspruch 8 oder 9 einschließt; und
einen Träger, der die Haftkleberschicht trägt.

## Revendications

1. Préparation à usage externe contenant :
un médicament anti-inflammatoire ; et
un ester d'acide gras et de polyglycérol lipophile ;
dans laquelle l'ester d'acide gras et de polyglycérol est un ester d'un polyglycérol ayant un degré moyen de polymérisation de 2 à 14 et d'un acide gras en C₁₄ à C₂₄ ; et
la teneur en eau de la préparation à usage externe ne dépassant pas 1,0 % en poids par rapport au poids total de la préparation à usage externe.

2. Préparation à usage externe selon la revendication 1, dans laquelle l'indice HLB de l'ester d'acide gras et de polyglycérol est d'au plus 9.

3. Préparation à usage externe selon la revendication 1 ou 2, dans laquelle l'ester d'acide gras et de polyglycérol est un ester d'un polyglycérol et d'un produit de condensation d'acides gras contenant des groupes hydroxyles.

4. Préparation à usage externe selon l'une quelconque des revendications 1 à 3, qui contient l'ester d'acide gras et de polyglycérol à raison de 0,01 à 10 % en poids par rapport au poids total.

5. Préparation à usage externe selon la revendication 1, dans laquelle le médicament anti-inflammatoire est au moins l'un parmi l'ensemble constitué par les analgésiques anti-inflammatoires non stéroïdiens, les médicaments antirhumatismaux modificateurs de la maladie, et les bloqueurs de cytokine.

6. Préparation à usage externe selon la revendication 5, dans laquelle l'analgésique anti-inflammatoire non stéroïdien est au moins l'un parmi l'ensemble constitué par l'indométhacine, le kétoprofène, le diclofénac, le flurbiprofène, le felbinac, l'ibuprofène, le suprofène, le tiaprofène, le loxoprofène, le célécoxib, le rofécoxib, le méloxicam et le valdécoxib.

7. Préparation à usage externe selon l'une quelconque des revendications 1 à 6, qui contient en outre l'un ou les deux parmi un sel d'acide édétique et/ou l'hydroxyde d'aluminium.

8. Préparation à usage externe selon l'une quelconque des revendications 1 à 7, qui contient en outre une base qui dissout ou disperse le composant pharmacologiquement actif et l'ester d'acide gras et de polyglycérol.

9. Préparation à usage externe selon la revendication 8, dans laquelle la base contient au moins une substance choisie dans l'ensemble constitué par un caoutchouc naturel, un caoutchouc d'isoprène synthétique, un polyisobutylène, un polyvinyléther, un polyuréthane, un polyisoprène, un polybutadiène, un copolymère de styrène/butadiène, un copolymère de styrène/isoprène, un copolymère séquencé de styrène/isoprène/styrène, les adhésifs acryliques sensibles à la pression et les adhésifs siliconés sensibles à la pression.

10. Timbre adhésif comprenant :
une couche adhésive sensible à la pression contenant une préparation à usage externe selon la revendication 8 ou 9 ; et
un support portant la couche adhésive sensible à la pression.
